(19) Europäisches Patentamt
European Patent Office
Office européen des brevets

(11) Veröffentlichungsnummer: **0 394 196 B1**

# (12) EUROPÄISCHE PATENTSCHRIFT

(45) Veröffentlichungstag der Patentschrift: **28.09.94**

(51) Int. Cl.[5]: **C09D 5/08**, C07D 277/74

(21) Anmeldenummer: **90810291.6**

(22) Anmeldetag: **10.04.90**

Die Akte enthält technische Angaben, die nach dem Eingang der Anmeldung eingereicht wurden und die nicht in dieser Patentschrift enthalten sind.

## (54) Antikorrosive Anstrichstoffe.

(30) Priorität: **20.04.89 CH 1501/89**

(43) Veröffentlichungstag der Anmeldung:
**24.10.90 Patentblatt 90/43**

(45) Bekanntmachung des Hinweises auf die Patenterteilung:
**28.09.94 Patentblatt 94/39**

(84) Benannte Vertragsstaaten:
**DE FR GB IT**

(56) Entgegenhaltungen:
**EP-A- 0 161 222**
**EP-A- 0 259 255**

(73) Patentinhaber: **CIBA-GEIGY AG**
**Klybeckstrasse 141**
**CH-4002 Basel (CH)**

(72) Erfinder: **Braig, Adalbert, Dr.**
**Zollstrasse 1/1**
**D-7858 Weil-Friedlingen (DE)**
Erfinder: **Phillips, Emyr, Dr.**
**1 Hill Top View,**
**Tingley**
**Wakefield, West Yorkshire (GB)**

**EP 0 394 196 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Anstrichstoffe, die als Korrosionsinhibitoren S-benzylierte Derivate des 2-Mercaptobenzthiazols enthalten.

2-Mercaptobenzthiazol und seine Salze sind als Korrosionsinhibitoren für Anstrichstoffe bekannt, z.B. aus der EP-A-3817. Es wurden auch schon verschiedene S-substituierte Derivate des Mercaptobenzthiazols für diese Verwendung vorgeschlagen, so z.B. Carbonsäurederivate (EP-A-128 862) und phenolische Derivate (EP-A-259 255). S-Benzylderivate des Mercaptobenzthiazols wurden als antikorrosive Zusätze für saure Metallbeizbäder vorgeschlagen (JP-A-85/141 879). Metallbeizbäder sind wässrige Lösungen von Säuren, und ein zugesetzter Korrosionsinhibitor muss an der Grenzfläche Wasser/Metall wirken. Demgegenüber muss ein Korrosionsinhibitor in Anstrichstoffen an der Grenzfläche Bindemittel/Metall wirken, was ganz andere physikalisch-chemische Eigenschaften voraussetzt. Es war daher überraschend zu finden, dass solche S-Benzylderivate von Mercaptobenzthiazol auch in Anstrichen eine hervorragende antikorrosive Wirkung auf den metallischen Untergrund haben. Diese Derivate zeichnen sich auch durch eine gute Löslichkeit in verschiedenen Anstrichstoffen aus und beeinträchtigen die Haftung des Anstriches auf dem metallischen Untergrund nicht.

Gegenstand der Erfindung ist daher ein Anstrichstoff, enthaltend als Korrosionsinhibitor mindestens eine Verbindung der Formel I

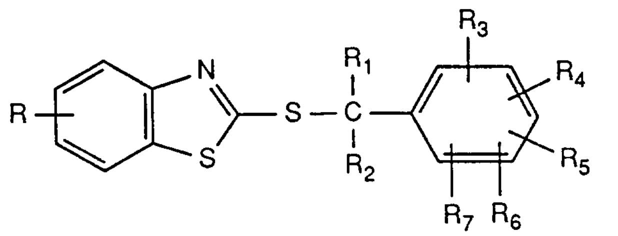

(I)

worin R Wasserstoff, Halogen, $C_1$-$C_{12}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $-NO_2$, -CN, -COOH, -COO($C_1$-$C_4$-Alkyl), -OH, $-NH_2$, $-NHR_8$, $-N(R_8)_2$, $-CONH_2$, $-CONHR_8$ oder $-CON(R_8)_2$ bedeutet,

$R_1$ Wasserstoff, $C_1$-$C_{12}$-Alkyl, Phenyl, durch Halogen, $C_1$-$C_4$-Alkyl, $C_1$-$C_4$-Alkoxy oder $-NO_2$ substituiertes Phenyl, Pyridyl, Thienyl oder Furyl bedeutet,

$R_2$ Wasserstoff, $C_1$-$C_4$-Alkyl oder Phenyl bedeutet,

$R_3$ und $R_4$ unabhängig voneinander H, $C_1$-$C_{20}$-Alkyl, $C_1$-$C_{20}$-Alkoxy, $-NO_2$, -CN, -COOH, -COO($C_1$-$C_4$-Alkyl), Phenyl, Halogen oder eine Gruppe der Formel II bedeuten

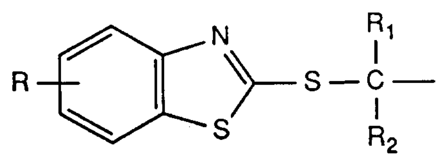

(II)

oder $R_3$ und $R_4$ zusammen eine Gruppe -CH = CH-CH = CH- bedeuten,

$R_5$, $R_6$ und $R_7$ Wasserstoff oder Halogen bedeuten,

$R_8$ $C_1$-$C_{12}$-Alkyl, $C_3$-$C_{12}$-Alkyl, das durch ein oder mehrere -O- unterbrochen ist, $C_5$-$C_8$-Cycloalkyl, Benzyl, Phenyl oder durch Halogen, $C_1$-$C_4$-Alkyl, $C_1$-$C_4$-Alkoxy oder $-NO_2$ substituiertes Phenyl bedeutet oder $-N(R_8)_2$ eine Pyrrolidino-, Piperidino- oder Morpholinogruppe bedeutet.

Wenn in Formel I R, $R_1$, $R_2$, $R_3$, $R_4$ oder $R_8$ Alkyl bedeutet, so kann dies unverzweigtes oder verzweigtes Alkyl sein. Beispiele hierfür sind Methyl, Ethyl, n-Propyl, i-Propyl, n-Butyl, sec-Butyl, i-Butyl, t-Butyl, n-Pentyl, sec-Pentyl, n-Hexyl, 2-Ethylbutyl, n-Octyl, 2-Ethylhexyl, i-Octyl, n-Decyl, n-Dodecyl, sec-Dodecyl, n-Tetradecyl, n-Hexadecyl, n-Octadecyl oder Eikosyl.

$R_8$ als durch -O- unterbrochenes Alkyl kann z.B. 2-Methoxyethyl, 2-Ethoxyethyl, 2-Butoxyethyl, 2-Methoxypropyl, 3,6-Dioxaheptyl, 3,6-Dioxaoctyl oder 3,6,9-Trioxadecyl sein.

R als Halogenalkyl kann z.B. Chlormethyl, Trichlormethyl, Trifluormethyl, Pentafluorethyl oder Nonafluorbutyl sein.

$R_8$ als Cycloalkyl kann z.B. Cyclopentyl, Cyclohexyl oder Cyclooctyl sein. R als Alkoxy kann z.B. Methoxy, Ethoxy, Isopropoxy, Butoxy, Hexyloxy, Octyloxy oder Dodecyloxy sein. $R_3$ und $R_4$ als Alkoxy können darüber hinaus auch z.B. Tetradecyloxy, Hexadecyloxy oder Octadecyloxy sein. R als Alkylthio kann z.B. Methylthio, Ethylthio, t-Butylthio, Octylthio oder Dodecylthio sein.

$R_1$ und $R_8$ als durch Halogen, $C_1$-$C_4$-Alkyl, $C_1$-$C_4$-Alkoxy oder $NO_2$ substituiertes Phenyl können z.B. Tolyl, Xylyl, Isopropylphenyl, t-Butylphenyl, Chlorphenyl, Dichlorphenyl, Fluorphenyl, Methoxyphenyl, Ethoxyphenyl, Butoxyphenyl oder Nitrophenyl sein.

Wenn $R_3$ und $R_4$ zusammen eine Gruppe -CH=CH-CH=CH- bedeuten, so bilden sie zusammen mit der Phenylgruppe, an die sie gebunden sind, eine Naphthylgruppe. Dies kann eine $\alpha$- oder $\beta$-Naphthylgruppe sein.

Bevorzugte Korrosionsinhibitoren sind solche der Formel I, worin R Wasserstoff, $C_1$-$C_4$-Alkyl, $CF_3$, $C_1$-$C_4$-Alkoxy, Halogen, $-NO_2$, -COOH oder -COO($C_1$-$C_4$-Alkyl) bedeutet,
$R_1$ Wasserstoff, $C_1$-$C_4$-Alkyl, Phenyl oder Tolyl bedeutet,
$R_2$ Wasserstoff, $C_1$-$C_4$-Alkyl oder Phenyl bedeutet,
$R_3$ und $R_4$ unabhängig voneinander H, $C_1$-$C_4$-Alkyl, $C_1$-$C_4$-Alkoxy, $-NO_2$, -CN, -COO($C_1$-$C_4$-Alkyl), Halogen oder eine Gruppe der Formel II bedeuten oder
$R_3$ und $R_4$ zusammen eine Gruppe -CH=CH-CH=CH- bedeuten und
$R_5$, $R_6$ und $R_7$ Wasserstoff oder Halogen bedeuten.

Besonders bevorzugt sind Korrosionsinhibitoren der Formel I, worin
R Wasserstoff, $C_1$-$C_4$-Alkyl, $C_1$-$C_4$-Alkoxy oder Halogen bedeutet,
$R_1$ Wasserstoff, $C_1$-$C_4$-Alkyl oder Phenyl bedeutet,
$R_2$ Wasserstoff oder Phenyl bedeutet,
$R_3$ und $R_4$ unabhängig voneinander H, $C_1$-$C_4$-Alkyl, $C_1$-$C_4$-Alkoxy, Halogen oder eine Gruppe der Formel II bedeuten oder $R_3$ und $R_4$ zusammen eine Gruppe -CH=CH-CH=CH- bedeuten und
$R_5$, $R_6$ und $R_7$ Wasserstoff oder Fluor bedeuten.

Bevorzugt sind Verbindungen der Formel I, worin R Wasserstoff ist, sowie solche Verbindungen der Formel I, worin $R_1$ Wasserstoff oder Methyl ist und $R_2$ Wasserstoff ist.

Beispiele für Verbindungen der Formel I sind:
2-Benzylthio-benzthiazol
2-(2-Chlorbenzylthio)-benzthiazol
2-(4-Chlorbenzylthio)-benzthiazol
2-(2-Brombenzylthio)-benzthiazol
2-(4-Brombenzylthio)-benzthiazol
2-(2-Fluorbenzylthio)-benzthiazol
2-(4-Fluorbenzylthio)-benzthiazol
2-(2,4-Dichlorbenzylthio)-benzthiazol
2-(2,4-Dibrombenzylthio)-benzthiazol
2-(2,3,4,5,6-Pentachlorbenzylthio)-benzthiazol
2-(2,3,4,5,6-Pentabrombenzylthio)benzthiazol
2-(2,3,4,5,6-Pentafluorbenzylthio)-benzthiazol
2-(2-Methylbenzylthio)-benzthiazol
2-(4-Methylbenzylthio)-benzthiazol
2-(2-Methoxybenzylthio)-benzthiazol
2-(4-Methoxybenzylthio)-benzthiazol
2-(2-Nitrobenzylthio)-benzthiazol
2-(4-Nitrobenzylthio)-benzthiazol
2-(2-Cyanobenzylthio)-benzthiazol
2-(4-Cyanobenzylthio)-benzthiazol
2-(Diphenylmethylthio)-benzthiazol
2-(Triphenylmethylthio)-benzthiazol
2-(1-Naphthylmethylthio)-benzthiazol
2-(2-Naphthylmethylthio)-benzthiazol
6-Chlor-2-(benzylthio)-benzthiazol
5-Ethoxy-2-(benzylthio)-benzthiazol.

Die Verbindungen der Formel I sind zum Teil bekannte und zum Teil neue Verbindungen. Bekannt sind solche Verbindungen der Formel I, worin R Wasserstoff, Chlor, Methyl, Methoxy oder Amino ist, $R_1$ und $R_2$ Wasserstoff sind, $R_3$ und $R_4$ Wasserstoff oder Chlor sind und $R_5$, $R_6$ und $R_7$ Wasserstoff sind.

Die Erfindung betrifft daher auch Verbindungen der Formel I

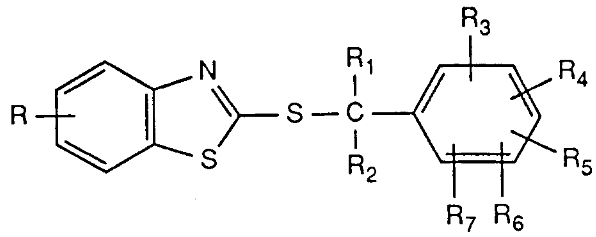

(I)

worin R Wasserstoff, Halogen, $C_1$-$C_{12}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $-NO_2$, -CN, -COOH, -COO($C_1$-$C_4$-Alkyl), -OH, $-NH_2$, $-NHR_8$, $-N(R_8)_2$, $-CONH_2$, $-CONHR_8$ oder $-CON(R_8)_2$ bedeutet,
$R_1$ Wasserstoff, $C_1$-$C_{12}$-Alkyl, Phenyl, durch Halogen, $C_1$-$C_4$-Alkyl, $C_1$-$C_4$-Alkoxy oder $-NO_2$ substituiertes Phenyl, Pyridyl, Thienyl oder Furyl bedeutet,
$R_2$ Wasserstoff, $C_1$-$C_4$-Alkyl oder Phenyl bedeutet,
$R_3$ und $R_4$ unabhängig voneinander H, $C_1$-$C_{20}$-Alkyl, $C_1$-$C_{20}$-Alkoxy, $-NO_2$, -CN, -COOH, -COO($C_1$-$C_4$-Alkyl), Phenyl, Halogen oder eine Gruppe der Formel II bedeuten

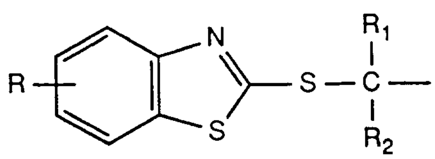

(II)

oder $R_3$ und $R_4$ zusammen eine Gruppe -CH = CH-CH = CH- bedeuten,
$R_5$, $R_6$ und $R_7$ Wasserstoff oder Halogen bedeuten,
$R_8$ $C_1$-$C_{12}$-Alkyl, $C_3$-$C_{12}$-Alkyl, das durch ein oder mehrere -O- unterbrochen ist, $C_5$-$C_8$-Cycloalkyl, Benzyl, Phenyl oder durch Halogen, $C_1$-$C_4$-Alkyl, $C_1$-$C_4$-Alkoxy oder $-NO_2$ substituiertes Phenyl bedeutet oder $-N(R_8)_2$ eine Pyrrolidino-, Piperidino- oder Morpholinogruppe bedeutet, mit Ausnahme der Verbindungen der Formel I, worin R Wasserstoff, Chlor, Methyl, Methoxy oder Amino ist, $R_1$ und $R_2$ Wasserstoff sind, $R_3$ und $R_4$ Wasserstoff oder Chlor sind und $R_5$, $R_6$ und $R_7$ Wasserstoff sind.

Bevorzugt sind darunter

a) Verbindungen der Formel I, worin $R_1$ Wasserstoff, $C_1$-$C_4$-Alkyl oder Phenyl ist und $R_2$ $C_1$-$C_4$-Alkyl oder Phenyl ist,

b) Verbindungen der Formel I, worin R $-CF_3$, $-NO_2$, -COOH oder -COO($C_1$-$C_4$-Alkyl) bedeutet und

c) Verbindungen der Formel I, worin $R_3$ und $R_4$ unabhängig voneinander $C_1$-$C_4$-Alkyl, $C_1$-$C_4$-Alkoxy, $-NO_2$, -CN, -Br, -F oder einen Rest der Formel II bedeuten oder $R_3$ und $R_4$ zusammen einen Rest -CH = CH-CH = CH- bedeuten.

Die Verbindungen können in an sich bekannter Weise hergestellt werden durch Umsetzung eines Natriummercaptides der Formel III mit einem Benzylhalogenid der Formel IV gemäss

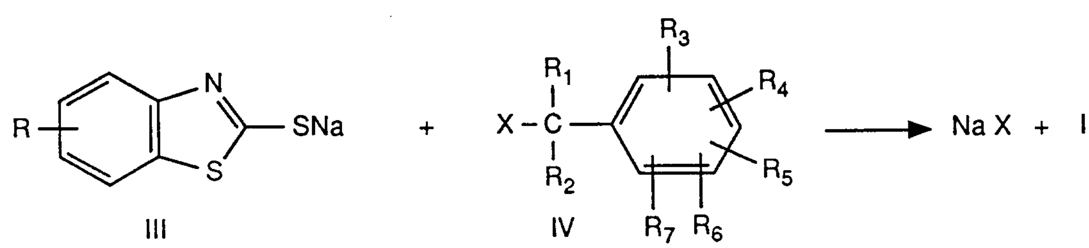


worin X Chlor oder Brom bedeutet. Die Umsetzung wird vorzugsweise in einem polaren Lösungsmittel ausgeführt, z.B. in Methanol, Ethanol, Isopropanol oder Dimethylformamid.

Die Verbindungen der Formel I sind wirksame Korrosionsinhibitoren in Anstrichstoffen. Anstrichstoffe sind z.B. Lacke, Farben oder Firnisse. Sie enthalten stets ein filmbildendes Bindemittel neben anderen fakultativen Komponenten.

Beispiele für Anstrichstoffe sind solche auf Basis eines Alkyd-, Acryl-, Melamin-, Polyurethan-, Epoxid- oder Polyesterharzes sowie von Gemischen solcher Harze. Weitere Beispiele für Bindemittel sind Vinylharze wie z.B. Polyvinylacetat, Polyvinylbutyral, Polyvinylchlorid und dessen Copolymere, Celluloseester, chlorierte Kautschuke, Phenolharze, Styrol-Butadien-Copolymere und trocknende Oele. Besonders bevorzugt sind Anstrichstoffe auf Basis eines aromatischen Epoxidharzes.

Beispiele für Anstrichstoffe mit speziellen Bindemitteln sind die folgenden:

1. Lacke auf Basis von kalt- oder heiss-vernetzbaren Alkyd-, Acrylat-, Polyester-, Epoxid- oder Melaminharzen oder Mischungen solcher Harze, gegebenenfalls mit Zusatz eines sauren Härtungskatalysators;
2. Zweikomponenten-Polyurethanlacke auf Basis von hydroxylgruppenhaltigen Acrylat-, Polyester- oder Polyetherharzen und aliphatischen oder aromatischen Polyisocyanaten;
3. Einkomponenten-Polyurethanlacke auf Basis von blockierten Polyisocyanaten, die während des Einbrennens deblockiert werden;
4. Zweikomponentenlacke auf Basis von (Poly)ketiminen und aliphatischen oder aromatischen Polyisocyanaten;
5. Zweikomponentenlacke auf Basis von (Poly)ketiminen und einem ungesättigten Acrylatharz oder einem Polyacetoacetatharz oder einem Methylacrylamidoglykolatmethylester;
6. Zweikomponentenlacke auf Basis von carboxyl- oder aminogruppenhaltigen Polyacrylaten und Polyepoxiden;
7. Zweikomponentenlacke auf Basis von anhydridgruppenhaltigen Acrylatharzen und einer Polyhydroxy- oder Polyaminokomponente;
8. Zweikomponentenlacke auf Basis von (Poly)oxazolidinen und anhydridgruppenhaltign Acrylatharzen oder ungesättigten Acrylatharzen oder aliphatischen oder aromatischen Polyisocyanaten.
9. Zweikomponentenlacke auf Basis von ungesättigten Polyacrylaten und Polymalonaten;
10. thermoplastische Polyacrylatlacke auf Basis von thermoplastischen Acrylatharzen oder fremdvernetzenden Acrylatharzen in Kombination mit veretherten Melaminharzen;
11. Lacksysteme auf Basis von siloxanmodifizierten Acrylatharzen.

Die Anstrichstoffe können pigmentiert oder unpigmentiert sein. Die Pigmente können anorganische oder organische Pigmente oder Metallpigmente sein. Metallpigmente, wie z.B. Aluminiumpigmente, werden dabei durch die Anwesenheit der Verbindungen der Formel I gegen Korrosion geschützt.

Die Anstrichstoffe können ein organisches Lösungsmittel enthalten oder lösungsmittelfrei sein oder wässrige Anstrichstoffe sein. Die Anstrichstoffe können auch strahlenhärtbar sein. In diesem Fall besteht das Bindemittel aus monomeren oder oligomeren Verbindungen, die ethylenische Doppelbindungen enthalten und durch Bestrahlung mit aktinischem Licht oder mit Elektronenstrahlen in eine vernetzte hochmolekulare Form übergehen.

Die Anstrichstoffe können weitere Zusätze enthalten, wie z.B. Füllstoffe, Verlaufhilfsmittel, Dispergiermittel, Thixotropiemittel, Haftverbesserer, Antioxidantien, Lichtschutzmittel oder Härtungskatalysatoren. Sie können auch andere bekannte Korrosionsschutzmittel enthalten, beispielsweise Korrosionsschutz-Pigmente, wie phosphat- oder borathaltige Pigmente oder Metalloxid-Pigmente, oder andere organische oder anorganische Korrosionsinhibitoren, z.B. Salze der Nitroisophthalsäure, Phosphorester, technische Amine oder substituierte Benztriazole.

Von Vorteil ist ferner der Zusatz von basischen Füllstoffen oder Pigmenten, die in bestimmten Bindemittelsystemen einen synergistischen Effekt auf die Korrosionsinhibierung bewirken. Beispiele für solche basische Füllstoffe und Pigmente sind Calcium- oder Magnesiumcarbonat, Zinkoxid, Zinkcarbonat, Zinkphosphat, Magnesiumoxid, Aluminiumoxid, Aluminiumphosphat oder Gemische davon. Beispiele für basische organische Pigmente sind solche auf Basis von Aminoanthrachinon.

Der Korrosionsinhibitor kann dem Anstrichstoff während dessen Herstellung zugesetzt werden, z.B. während der Pigmentverteilung durch Mahlen, oder man löst den Inhibitor in einem Lösungsmittel vor und rührt die Lösung in das Ueberzugsmittel ein. Man verwendet den Inhibitor zweckmässig in einer Menge von 0,1 bis 20 Gew.%, vorzugsweise 0,5 bis 5 Gew.%, bezogen auf den Feststoffgehalt des Anstrichstoffes. In bestimmten Fällen kann der Zusatz von mehreren Verbindungen der Formel I vorteilhaft sein.

Bevorzugt werden die Anstrichstoffe als Primer für metallische Substrate, wie z.B. für Eisen, Stahl, Kupfer, Zink oder Aluminium, verwendet. Bevorzugt werden die Anstrichstoffe in wässrigen Systemen verwendet, insbesondere als kathodisch abscheidbare Elektrophoreselacke.

Die Anstrichstoffe können nach den üblichen Verfahren auf das Substrat aufgebracht werden, z.B. durch Sprühen, Tauchen, Streichen oder durch Elektroabscheidung, wie z.B. durch kathodische Tauchlackierung.

Oft werden mehrere Schichten aufgetragen. Die Korrosionsinhibitoren werden in erster Linie der Grundschicht zugegeben, da sie vor allem an der Grenze Metall-Anstrich wirken. Man kann aber die Inhibitoren auch zusätzlich der Deckschicht oder Zwischenschicht zugeben, wo sie als Depot zur Verfügung stehen. Je nachdem, ob das Bindemittel ein physikalisch trocknendes oder hitze- oder strahlenhärtbares Harz ist, erfolgt die Härtung bei Raumtemperatur oder durch Erwärmen (Einbrennen) oder durch Bestrahlung.

Die folgenden Beispiele beschreiben die Herstellung einzelner Verbindungen der Formel I und deren Verwendung. Darin bedeuten Teile Gewichtsteile und % Gewichts-%. Die Temperaturen werden in °C angegeben.

Beispiel 1: 2-(Benzylthio)-benzthiazol

Eine Lösung von 28,1 g (0,2 Mol) Benzylchlorid in 50 ml Ethanol wird langsam unter Rühren zu einer Lösung von 33,4 g (0,2 Mol) 2-Mercaptobenzthiazol und 8 g (0,2 Mol) NaOH in 150 ml Ethanol und 25 ml Wasser gegeben. Die Lösung wird 2,5 h unter Rückfluss erwärmt. Nach dem Abkühlen wird die Lösung filtriert und eingedampft. Es hinterbleiben 35,1 g eines gelblichen Feststoffes, der bei 39 - 40° schmilzt.
$^1$H-NMR ($CDCl_3$) $\delta$ 4,41 (2H), $\delta$ 6,98 - 7,8 (9H) ppm.

Beispiele 2-12: Herstellung analoger Benzthiazole

Analog Beispiel 1 werden die folgenden Verbindungen hergestellt:

| Beispiel | Formel | Smp. | $^1$H-NMR ($CDCl_3$) |
|---|---|---|---|
| 2 | 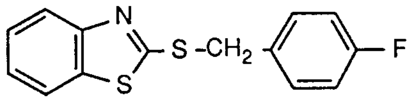 | 183-5° | 4,40 (2H), 6,65-7,85 8(H) ppm |
| 3 | 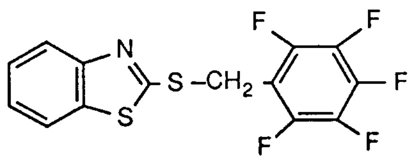 | 51-2° | 4,61 (2H), 7,05-7,97 (4H) ppm |
| 4 | 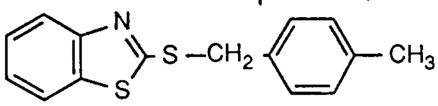 | 49-51° | 2,25 (3H), 4,50 (2H), 7,0-7,98 (8H) ppm |
| 5 | 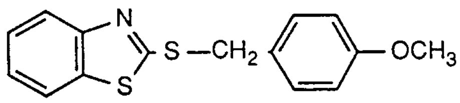 | 67-8° | 3,75 (3H), 4,50 (2H), 6,77-7,92 (8H) ppm |
| 6 | 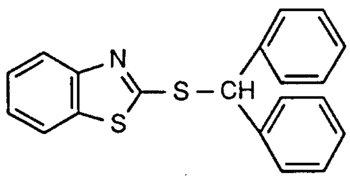 | 97-8° | 6,30 (1H), 7,10-7,90 (14H) ppm |
| 7 | 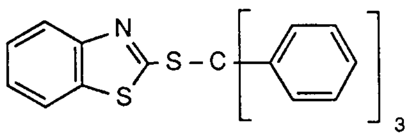 | 101-2° | 7,14-7,55 (19H) ppm |
| 8 | 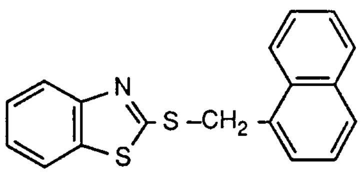 | 75-6° | 4,92 (2H), 6,99-7,99 (11H) ppm |
| 9 | 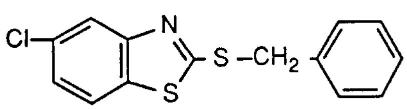 | 72-3° | 4,35 (2H), 6,82-7,63 (8H) ppm |
| 10 | 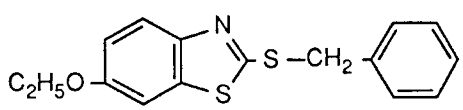 | 76-8° | 1,15 (3H), 3,80 (2H) 4,36 (2H), 6,60-7,59 (8H) |
| 11 | 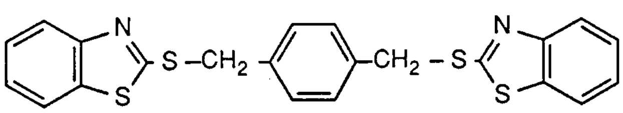 | 104-6° | 4,59 (4H), 7,21-8,0 (12H) ppm |
| 12 | 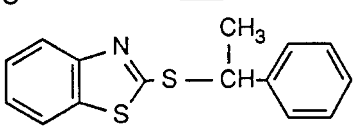 | Oel | 1,55 (3H), 4,76 (1H), 6,68-7,65 (9H) ppm |

Beispiel 13: Antikorrosiver Alkydharz-Lack

Zur Bereitung des Lackes werden folgende Komponenten gemischt:
40 Teile Alphthalat® AM 380 (60 %ige Xylol-Lösung) Alkydharz der Fa. Reichhold Albert Chemie AG

10 Teile Eisenoxidrot 225 der Fa. Bayer AG
13,6 Teile Talkum (mikronisiert)
13 Teile Mikronisiertes Calciumcarbonat (Millicarb®, Plüss-Staufer AG)
0,3 Teile Hautverhütungsmittel Luaktin® (BASF)
0,6 Teile 8 %ige Cobaltnaphthenat-Lösung
24,7 Teile Xylol/Propylenglykolmonomethylether-Gemisch 6:40.

Die in der folgenden Tabelle angegebenen Korrosionsinhibitoren werden in einem Teil des Lösungsmittels vorgelöst und dem Lack zugegeben. Der Lack wird 7 Tage mit Glasperlen gemahlen bis zu einer Pigment- und Füllstoffkorngrösse von unter 15 µm.

Der Lack wird auf sandgestrahlte Stahlbleche von 7 x 13 cm aufgespritzt in einer Schichtdicke, die nach dem Trocknen ca. 50 µm beträgt. Nach 7 Tagen Trocknung bei Raumtemperatur werden die Proben 60 Minuten bei 60 °C nachgehärtet.

In die gehärtete Lackoberfläche werden zwei kreuzförmige Schnitte von 4 cm Länge bis zum Metall eingeschnitten mit Hilfe eines Bonder-Kreuzschnittgerätes. Zum Schutze der Kanten wird auf diese ein Kantenschutzmittel (Icosit® 255) aufgebracht.

Die Proben werden nunmehr einem Salzsprühtest gemäss ASTM B 117 mit einer Dauer von 600 Stunden unterworfen. Nach jeweils 200 Stunden Bewitterung wird der Zustand des Anstriches beurteilt, und zwar der Blasengrad (gemäss DIN 53 209) am Kreuzschnitt und auf der lackierten Fläche sowie der Rostgrad (gemäss DIN 53210) auf der ganzen Fläche.

Nach Ende des Tests wird der Anstrich durch Behandlung mit konzentrierter Natronlauge entfernt und die Korrosion des Metalls am Kreuzschnitt (gemäss DIN 53 167) sowie über die restliche Fläche beurteilt. Die Beurteilung erfolgtjeweils in einer 6-Stufen-Skala. Die Summe der Bewertung des Anstriches und der Bewertung der Metalloberfläche ergibt den Korrosionsschutzwert KS. Je höher dieser ist, desto wirkungsvoller ist der getestete Inhibitor.

| Korrosionsinhibitor | Menge | Bewertung | Bewertung | KS |
|---|---|---|---|---|
| ohne | - | 1,8 | 0,6 | 2,4 |
| Beispiel 1 | 2% | 4,9 | 3,2 | 8,1 |
| Beispiel 2 | 2% | 3,7 | 4,3 | 8,0 |
| Beispiel 3 | 2% | 5,3 | 4,6 | 9,9 |
| Beispiel 4 | 2% | 2,6 | 2,6 | 5,2 |
| Beispiel 5 | 2% | 3,3 | 2,5 | 5,8 |
| Beispiel 6 | 2% | 3,8 | 3,5 | 7,3 |
| Beispiel 7 | 2% | 3,4 | 3,7 | 7,1 |
| Beispiel 8 | 2 % | 3,2 | 3,0 | 6,2 |
| Beispiel 9 | 2% | 4,5 | 4,1 | 8,6 |
| Beispiel 10 | 2% | 3,9 | 3,3 | 7,2 |
| Beispiel 11 | 2% | 3,2 | 3,0 | 6,2 |
| Beispiel 12 | 2% | 5,1 | 4,0 | 9,1 |

Beispiel 14: Antikorrosiver Elektrotauchlack

217,8 g eines Elektrotauchlackes werden mit 1,5 g Propylenglykolmonophenylether, 7,5 g Milchsäure (88%ig) und 1,5 g eines nichtionischen Netzmittels (X-Blend, Du Pont) unter Rühren vermischt. Der Elektrotauchlack ist eine Lösung eines aminogruppen- und hydroxylgruppenhaltigen aromatischen Epoxidharzes und enthält als Vernetzungsmittel ein verkapptes Diisocyanat. Der Lack ist ein Produkt der Firma Du Pont de Nemour und enthält ca. 36 % Feststoffe.

Zu obiger Mischung werden 10 g Dibutylzinndilaurat als Vernetzungskatalysator und 4 g des Korrosionsinhibitors des Beispieles 12 (2-(1-Phenylethylthio)-benzthiazol) zugegeben und bis zur homogenen Lösung gerührt. Dies entspricht einem Gehalt von 4,2 % Korrosionsinhibitor, bezogen auf den Festkörper.

Unter Rühren gibt man zunächst 76 g Wasser zu. Nach 20 Minuten werden weitere 76 g Wasser zugegeben. Nach 30 Minuten Rühren werden weitere 48 g Wasser zugegeben. Die entstandene Emulsion wird 48 Stunden gerührt und dann mit Wasser auf ein Volumen von 1000 ml aufgefüllt. Das so erhaltene Bad hat einen Festkörpergehalt von ca. 10 %. Sein pH-Wert ist 4,9, die Leitfähigkeit beträgt 2000 µS.

In dieses Bad werden Stahlbleche von 15 x 7,5 cm als Kathode getaucht. Bei einer Badtemperatur von 29 °C wird zur Abscheidung des Lackes 120 Sekunden eine Spannung von 220 Volt angelegt. Die Bleche

werden dann mit Wasser gespült, kurz getrocknet und 30 Minuten bei 176 °C eingebrannt. Der resultierende gehärtete Lackfilm hat eine Dicke von ca. 24 µm.

Zur Korrosionsprüfung wird die Oberfläche der Bleche mit einem Schnitt von 70 x 0,5 mm versehen und einem GM Scab-Test (TM 54-26) unterworfen.

Ein Prüfzyklus besteht in 15 Minuten Immersion der Proben in einer 5%igen wässrigen NaCl-Lösung, anschliessend 75 Minuten Lagerung bei Raumtemperatur und 22,5 Stunden in einer Feuchtklimakammer (Humidity Cabinet, Hot Pack Model 417522) bei 60 °C und 85 % relativer Feuchtigkeit. Nach 5 solcher Tageszyklen werden die Proben nochmals 2 Tage in der Feuchtklimakammer gelagert.

Anschliessend werden die Bleche mit Wasser gespült. Der durch Korrosion nicht mehr fest haftende Lack wird mechanisch entfernt und die durchschnittliche Breite der Korrosionszone am Schnitt gemessen.

Die durchschnittliche Breite der Korrosionszone ist bei den Proben, die den Korrosionsinhibitor enthalten, 7 mm. Bei Kontrollproben ohne Korrosionsinhibitor ist die Korrosionszone durchschnittlich 31 mm breit.

## Patentansprüche

**1.** Anstrichstoff, enthaltend als Korrosionsinhibitor mindestens eine Verbindung der Formel I

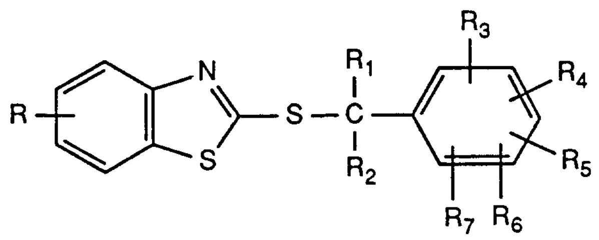

(I)

worin R Wasserstoff, Halogen, $C_1$-$C_{12}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $-NO_2$, -CN, -COOH, -COO($C_1$-$C_4$-Alkyl), -OH, $-NH_2$, $-NHR_8$, $-N(R_8)_2$, $-CONH_2$, $-CONHR_8$ oder $-CON(R_8)_2$ bedeutet,
$R_1$ Wasserstoff, $C_1$-$C_{12}$-Alkyl, Phenyl, durch Halogen, $C_1$-$C_4$-Alkyl, $C_1$-$C_4$-Alkoxy oder $-NO_2$ substituiertes Phenyl, Pyridyl, Thienyl oder Furyl bedeutet,
$R_2$ Wasserstoff, $C_1$-$C_4$-Alkyl oder Phenyl bedeutet,
$R_3$ und $R_4$ unabhängig voneinander H, $C_1$-$C_{20}$-Alkyl, $C_1$-$C_{20}$-Alkoxy, $-NO_2$, -CN, -COOH, -COO($C_1$-$C_4$-Alkyl), Phenyl, Halogen oder eine Gruppe der Formel II bedeuten

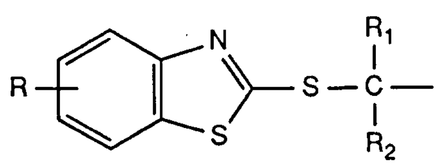

(II)

oder $R_3$ und $R_4$ zusammen eine Gruppe -CH=CH-CH=CH- bedeuten,
$R_5$, $R_6$ und $R_7$ Wasserstoff oder Halogen bedeuten,
$R_8$ $C_1$-$C_{12}$-Alkyl, $C_3$-$C_{12}$-Alkyl, das durch ein oder mehrere -O- unterbrochen ist, $C_5$-$C_8$-Cycloalkyl, Benzyl, Phenyl oder durch Halogen, $C_1$-$C_4$-Alkyl, $C_1$-$C_4$-Alkoxy oder $-NO_2$ substituiertes Phenyl bedeutet oder $-N(R_8)_2$ eine Pyrrolidino-, Piperidino- oder Morpholinogruppe bedeutet.

**2.** Anstrichstoff gemäss Anspruch 1, enthaltend mindestens eine Verbindung der Formel I, worin
R Wasserstoff, $C_1$-$C_4$-Alkyl, $CF_3$, $C_1$-$C_4$-Alkoxy, Halogen, $-NO_2$, -COOH oder -COO($C_1$-$C_4$-Alkyl) bedeutet, $R_1$ Wasserstoff, $C_1$-$C_4$-Alkyl, Phenyl oder Tolyl bedeutet,
$R_2$ Wasserstoff, $C_1$-$C_4$-Alkyl oder Phenyl bedeutet,
$R_3$ und $R_4$ unabhängig voneinander H, $C_1$-$C_4$-Alkyl, $C_1$-$C_4$-Alkoxy, $-NO_2$, -CN, -COO($C_1$-$C_4$-Alkyl), Halogen oder eine Gruppe der Formel II bedeuten oder
$R_3$ und $R_4$ zusammen eine Gruppe -CH=CH-CH=CH- bedeuten und
$R_5$, $R_6$ und $R_7$ Wasserstoff oder Halogen bedeuten.

**3.** Anstrichstoff gemäss Anspruch 1, enthaltend mindestens eine Verbindung der Formel I, worin R Wasserstoff, $C_1$-$C_4$-Alkyl, $C_1$-$C_4$-Alkoxy oder Halogen bedeutet, $R_1$ Wasserstoff, $C_1$-$C_4$-Alkyl oder Phenyl bedeutet,
$R_2$ Wasserstoff oder Phenyl bedeutet,
$R_3$ und $R_4$ unabhängig voneinander H, $C_1$-$C_4$-Alkyl, $C_1$-$C_4$-Alkoxy, Halogen oder eine Gruppe der Formel II bedeuten oder
$R_3$ und $R_4$ zusammen eine Gruppe -CH = CH-CH = CH- bedeuten und
$R_5$, $R_6$ und $R_7$ Wasserstoff oder Fluor bedeuten.

**4.** Anstrichstoff gemäss Anspruch 1, enthaltend mindestens eine Verbindung der Formel I, worin R Wasserstoff ist.

**5.** Anstrichstoff gemäss Anspruch 1, enthaltend mindestens eine Verbindung der Formel I, worin $R_1$ Wasserstoff oder Methyl ist und $R_2$ Wasserstoff ist.

**6.** Anstrichstoff gemäss Anspruch 1, enthaltend 0,5 bis 5 Gew.-%, bezogen auf den Feststoffgehalt des Anstrichstoffes von mindestens einer Verbindung der Formel I.

**7.** Anstrichstoff gemäss Anspruch 1, welcher ein Primer für metallische Substrate ist.

**8.** Anstrichstoff gemäss Anspruch 7, welcher ein Primer auf Eisen, Stahl, Kupfer, Zink oder Aluminium ist.

**9.** Anstrichstoff gemäss Anspruch 1, welcher ein wässriger Anstrichstoff ist.

**10.** Anstrichstoff gemäss Anspruch 9, welcher ein kathodisch abscheidbarer Anstrichstoff ist.

**11.** Anstrichstoff gemäss Anspruch 1 auf der Basis eines Alkyd-, Acryl-, Melamin-, Polyurethan-, Epoxid- oder Polyesterharzes oder einer Mischung solcher Harze.

**12.** Verbindungen der Formel I

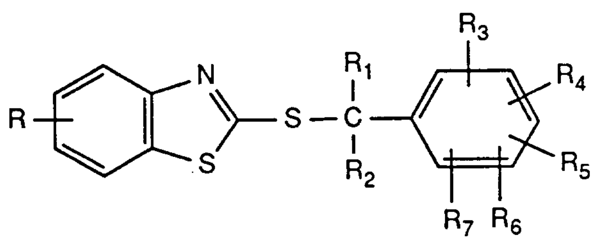

(I)

worin R Wasserstoff, Halogen, $C_1$-$C_{12}$-Alkyl, $C_1$-$C_4$-Halogenalkyl, $C_1$-$C_{12}$-Alkoxy, $C_1$-$C_{12}$-Alkylthio, Phenylthio, Benzylthio, $C_1$-$C_{12}$-Alkylsulfonyl, Phenyl, $-NO_2$, -CN, -COOH, -COO($C_1$-$C_4$-Alkyl), -OH, $-NH_2$, $-NHR_8$, $-N(R_8)_2$, $-CONH_2$, $-CONHR_8$ oder $-CON(R_8)_2$ bedeutet,
$R_1$ Wasserstoff, $C_1$-$C_{12}$-Alkyl, Phenyl, durch Halogen, $C_1$-$C_4$-Alkyl, $C_1$-$C_4$-Alkoxy oder $-NO_2$ substituiertes Phenyl, Pyridyl, Thienyl oder Furyl bedeutet,
$R_2$ Wasserstoff, $C_1$-$C_4$-Alkyl oder Phenyl bedeutet,
$R_3$ und $R_4$ unabhängig voneinander H, $C_1$-$C_{20}$-Alkyl, $C_1$-$C_{20}$-Alkoxy, $-NO_2$, -CN, -COOH, -COO($C_1$-$C_4$-Alkyl), Phenyl, Halogen oder eine Gruppe der Formel II bedeuten

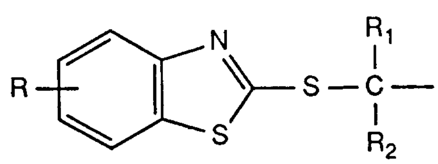

(II)

oder $R_3$ und $R_4$ zusammen eine Gruppe -CH = CH-CH = CH- bedeuten,

$R_5$, $R_6$ und $R_7$ Wasserstoff oder Halogen bedeuten,
$R_8$ $C_1$-$C_{12}$-Alkyl, $C_3$-$C_{12}$-Alkyl, das durch ein oder mehrere -O- unterbrochen ist, $C_5$-$C_8$-Cycloalkyl, Benzyl, Phenyl oder durch Halogen, $C_1$-$C_4$-Alkyl, $C_1$-$C_4$-Alkoxy oder $-NO_2$ substituiertes Phenyl bedeutet oder $-N(R_8)_2$ eine Pyrrolidino-, Piperidino- oder Morpholinogruppe bedeutet, mit Ausnahme der Verbindungen der Formel I, worin R Wasserstoff, Chlor, Methyl, Methoxy oder Amino ist, $R_1$ und $R_2$ Wasserstoff sind, $R_3$ und $R_4$ Wasserstoff oder Chlor sind und $R_5$, $R_6$ und $R_7$ Wasserstoff sind.

**13.** Verbindungen gemäss Anspruch 12 der Formel I, worin $R_1$ Wasserstoff, $C_1$-$C_4$-Alkyl oder Phenyl ist und $R_2$ $C_1$-$C_4$-Alkyl oder Phenyl ist.

**14.** Verbindungen gemäss Anspruch 12 der Formel I, worin R $-CF_3$, $-NO_2$, -COOH oder -COO($C_1$-$C_4$-Alkyl) bedeutet.

**15.** Verbindungen gemäss Anspruch 12 der Formel I, worin $R_3$ und $R_4$ unabhängig voneinander $C_1$-$C_4$-Alkyl, $C_1$-$C_4$-Alkoxy, $-NO_2$, -CN, -Br, -F oder einen Rest der Formel II bedeuten oder $R_3$ und $R_4$ zusammen einen Rest -CH = CH-CH = CH- bedeuten.

## Claims

**1.** A surface coating containing, as corrosion inhibitor, at least one compound of formula I:

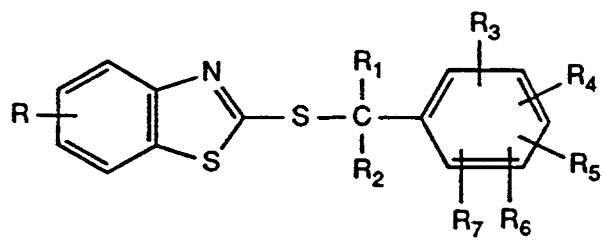

(I)

in which R is hydrogen, halogen, $C_1$-$C_{12}$alkyl, $C_1$-$C_4$haloalkyl, $C_1$-$C_{12}$alkoxy, $C_1$-$C_{12}$alkylthio, phenylthio, bensylthio, $C_1$-$C_{12}$alkylsulfonyl, phenyl, $-NO_2$, -CN, -COOH, -COO($C_1$-$C_4$alkyl), -OH, $-NH_2$, $-NHR_8$, $-CONH_2$, $-CONHR_8$ or $-CON(R_8)_2$, $R_1$ is hydrogen, $C_1$-$C_{12}$alkyl, phenyl, phenyl substituted by halogen, $C_1$-$C_4$alkyl, $C_1$-$C_4$alkoxy or $-NO_2$, pyridyl, thienyl or furyl, $R_2$ is hydrogen, $C_1$-$C_4$alkyl or phenyl, $R_3$ and $R_4$ independently of one another are H, $C_1$-$C_{20}$alkyl, $C_1$-$C_{20}$alkoxy, $-NO_2$, -CN, -COOH, -COO($C_1$-$C_4$alkyl),phenyl, halogen or a group of formula II:

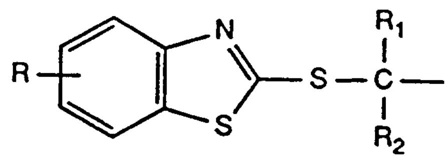

(II)

or $R_3$ and $R_4$ together are a group -CH = CH-CH = CH-, $R_5$, $R_6$ and $R_7$ are hydrogen or halogen and $R_8$ is $C_1$-$C_{12}$alkyl, $C_3$-$C_{12}$alkyl interrupted by one or more -O-, $C_5$-$C_8$cycloalkyl, benzyl, phenyl or phenyl substituted by halogen, $C_1$-$C_4$alkyl, $C_1$-$C_4$alkoxy or $-NO_2$, or $-N(R_8)_2$ is a pyrrolidino, piperidino or morpholino group.

**2.** A surface coating according to claim 1 containing at least one compound of formula I in which R is hydrogen, $C_1$-$C_4$alkyl, $CF_3$, $C_1$-$C_4$alkoxy, halogen, $-NO_2$, -COOH or -COO($C_1$-$C_4$alkyl), $R_1$ is hydrogen, $C_1$-$C_4$-alkyl, phenyl or tolyl, $R_2$ is hydrogen, $C_1$-$C_4$alkyl or phenyl, $R_3$ and $R_4$ independently of one another are H, $C_1$-$C_4$alkyl, $C_1$-$C_4$alkoxy, $-NO_2$, -CN, -COO($C_1$-$C_4$alkyl), halogen or a group of formula II, or $R_3$ and $R_4$ together are a group -CH = CH-CH = CH-, and $R_5$, $R_6$ and $R_7$ are hydrogen or halogen.

**3.** A surface coating according to claim 1 containing at least one compound of formula I in which R is hydrogen, $C_1$-$C_4$alkyl, $C_1$-$C_4$alkoxy or halogen, $R_1$ is hydrogen, $C_1$-$C_4$alkyl or phenyl, $R_2$ is hydrogen or phenyl, $R_3$ and $R_4$ independently of one another are H, $C_1$-$C_4$alkyl, $C_1$-$C_4$alkoxy, halogen or a group of formula II, or $R_3$ and $R_4$ together are a group -CH=CH-CH=CH-, and $R_5$, $R_6$ and $R_7$ are hydrogen or fluorine.

**4.** A surface coating according to claim 1 containing at least one compound of formula I in which R is hydrogen.

**5.** A surface coating according to claim 1 containing at leat one compound of formula I in which $R_1$ is hydrogen or methyl and $R_2$ is hydrogen.

**6.** A surface coating according to claim 1 containing 0.5 to 5% by weight, based on the solids content of the surface coating, of at least one compound of formula I.

**7.** A surface coating according to claim 1 which is a primer for metallic substrates.

**8.** A surface coating according to claim 7 which is a primer for iron, steel, copper, zinc or aluminium.

**9.** A surface coating according to claim 1 which is water-based.

**10.** A surface coating according to claim 9 which can be deposited cathodically.

**11.** A surface coating according to claim 1 based on an alkyd, acrylic, melamine, polyurethane, epoxy or polyester resin or a mixture of such resins.

**12.** A compound of formula I:

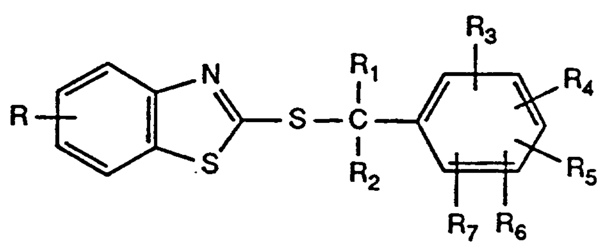

(I)

in which R is hydrogen, halogen, $C_1$-$C_{12}$alkyl, $C_1$-$C_4$haloalkyl, $C_1$-$C_{12}$alkoxy, $C_1$-$C_{12}$alkylthio, phenylthio, benzylthio, $C_1$-$C_{12}$alkylsulfonyl, phenyl, $-NO_2$, -CN, -COOH, -COO($C_1$-$C_4$alkyl), -OH, $NH_2$, $-NHR_8$, $-N(R_8)_2$, $-CONH_2$, $-CONHR_8$ or $-CON(R_8)_2$, $R_1$ is hydrogen, $C_1$-$C_{12}$alkyl, phenyl, phenyl substituted by halogen, $C_1$-$C_4$alkyl, $C_1$-$C_4$alkoxy or $-NO_2$, pyridyl, thienyl or furyl, $R_2$ is hydrogen, $C_1$-$C_4$alkyl or phenyl, $R_3$ and $R_4$ independently of one another are H, $C_1$-$C_{20}$alkyl, $C_1$-$C_{20}$alkoxy, $-NO_2$, -CN, -COOH, -COO($C_1$-$C_4$alkyl), phenyl, halogen or a group of formula II:

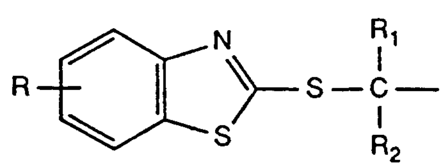

(II)

or $R_3$ and $R_4$ together are a group -CH=CH-CH=CH-, $R_5$, $R_6$ and $R_7$ are hydrogen or halogen and $R_8$ is $C_1$-$C_{12}$alkyl, $C_3$-$C_{12}$alkyl interrupted by one or more -O-, $C_5$-$C_8$cycloalkyl, benzyl, phenyl or phenyl substituted by halogen, $C_1$-$C_4$alkyl, $C_1$-$C_4$alkoxy or $-NO_2$, or $-N(R_8)_2$ is a pyrrolidino, piperidino or morpholino group, with the exception of the compounds of formula I in which R is hydrogen, chlorine, methyl, methoxy or amino, $R_1$ and $R_2$ are hydrogen, $R_3$ and $R_4$ are hydrogen or chlorine and $R_5$, $R_6$ and $R_7$ are hydrogen.

**13.** A compound according to claim 12 of formula I in which $R_1$ is hydrogen, $C_1$-$C_4$alkyl or phenyl and $R_2$ is $C_1$-$C_4$alkyl or phenyl.

**14.** A compound according to claim 12 of formula I in which R is $-CF_3$, $-NO_2$, -COOH or -COO($C_1$-$C_4$alkyl).

**15.** A compound according to claim 12 of formula I in which $R_3$ and $R_4$ independently of one another are $C_1$-$C_4$alkyl, $C_1$-$C_4$alkoxy, $-NO_2$, -CN, -Br, -F or a radical of formula II, or $R_3$ and $R_4$ together are a radical -CH = CH-CH = CH-.

## Revendications

**1.** Composition de revêtement, contenant comme inhibiteur de corrosion au moins un composé de Formule I

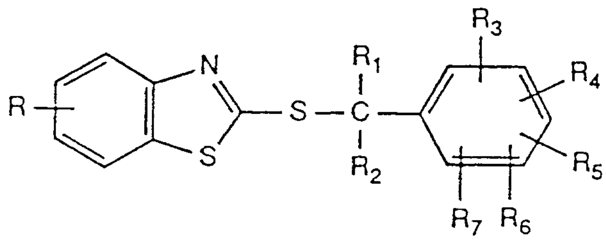

(I)

où R est hydrogène, un halogène ou un radical alkyle en $C_1$-$C_{12}$, halogénalkyle en $C_1$-$C_4$, alcoxy en $C_1$-$C_{12}$, alkylthio en $C_1$-$C_{12}$, phénylthio, benzylthio, alkylsulfonyle en $C_1$-$C_{12}$, phényle, $-NO_2$, -CN, -COOH, -COO(alkyle en $C_1$-$C_4$), -OH, $-NH_2$, $-NHR_8$, $-N(R_8)_2$, $-CONH_2$, $-CONHR_8$ ou $-CON(R_8)_2$,

$R_1$ est un hydrogène ou un radical alkyle en $C_1$-$C_{12}$, phényle, ou encore phényle substitué par des substituants halogéno, alkyle en $C_1$-$C_4$, alcoxy en $C_1$-$C_4$ ou $-NO_2$, ou encore pyridyle, thiényle ou furyle,

$R_2$ est un hydrogène ou un radical alkyle en $C_1$-$C_4$ ou phényle,

$R_3$ et $R_4$, indépendamment l'un de l'autre, sont chacun H ou un radical alkyle en $C_1$-$C_{20}$, alcoxy en $C_1$-$C_{20}$, $-NO_2$, -CN, -COOH, -COO(alkyle en $C_1$-$C_4$), phényle, halogéno, ou un groupe de Formule II

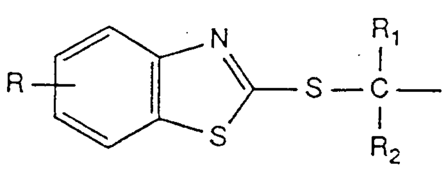

(II)

ou encore $R_3$ et $R_4$ forment ensemble un groupe -CH = CH-CH = CH-,

$R_5$, $R_6$ et $R_7$ sont chacun un hydrogène ou un halogène,

$R_8$ est un radical alkyle en $C_1$-$C_{12}$, alkyle en $C_3$-$C_{12}$ interrompu par un ou plusieurs oxygènes, cycloalkyle en $C_5$-$C_8$, benzyle, phényle, ou encore phényle substitués par des substituants halogéno, alkyle en $C_1$-$C_4$, alcoxy en $C_1$-$C_4$ ou $-NO_2$, ou encore $-N(R_8)_2$ est un groupe pyrrolidino, pipéridino ou morpholino

**2.** Composition de revêtement selon la revendication 1, contenant au moins un compose de Formule I, où

R est un hydrogène ou un radical alkyle en $C_1$-$C_4$, $CF_3$, alcoxy en $C_1$-$C_4$, halogéno, $-NO_2$, -COOH ou -COO(alkyle en $C_1$-$C_4$),

$R_1$ est un hydrogène ou un radical alkyle en $C_1$-$C_4$, phényle ou tolyle,

$R_2$ est un hydrogène ou un radical alkyle en $C_1$-$C_4$ ou phényle,

$R_3$ et $R_4$, indépendamment l'un de l'autre sont chacun H ou un radical alkyle en $C_1$-$C_4$, alcoxy en $C_1$-$C_4$, $-NO_2$, -CN, -COO(alkyle en $C_1$-$C_4$), halogéno, ou un groupe de Formule II, ou bien

$R_3$ et $R_4$ forment ensemble un groupe -CH = CH-CH = CH-, et

$R_5$, $R_6$ et $R_7$ sont chacun un hydrogène ou un halogène.

**3.** Composition de revêtement selon la revendication 1, contenant au moins un compose de Formule I, où
R est un hydrogène ou un radical alkyle en $C_1$-$C_4$, alcoxy en $C_1$-$C_4$ ou halogéno,
$R_1$ est un hydrogène ou un radical alkyle en $C_1$-$C_4$ ou phényle,
$R_2$ est un hydrogène ou le radical phényle,
$R_3$ et $R_4$, indépendamment l'un de l'autre sont chacun H ou un radical alkyle en $C_1$-$C_4$, alcoxy en $C_1$-$C_4$, halogéno, ou encore un groupe de Formule II, ou encore $R_3$ et $R_4$ forment ensemble un groupe -CH = CH-CH = CH-, et
$R_5$, $R_6$ et $R_7$ sont chacun un hydrogène ou le fluor

**4.** Composition de revêtement selon la revendication 1, contenant au moins un composé de Formule I dans laquelle R est un hydrogène.

**5.** Composition de revêtement selon la revendication 1, contenant au moins un composé de Formule I dans laquelle $R_1$ est un hydrogène ou le radical méthyle, et $R_2$ est un hydrogène.

**6.** Composition de revêtement selon la revendication 1, contenant de 0,5 à 5 % en poids, par rapport à l'extrait sec de la composition de revêtement, d'au moins un composé de Formule I.

**7.** Composition de revêtement selon la revendication 1, qui est un primaire pour subjectiles métalliques.

**8.** Composition de revêtement selon la revendication 7, qui est un primaire sur fer, acier, cuivre, zinc ou aluminium.

**9.** Composition de revêtement selon la revendication 1, qui est une composition de revêtement aqueuse.

**10.** Composition de revêtement selon la revendication 9, qui est une composition de revêtement pouvant être déposée par voie cathodique.

**11.** Composition de revêtement selon la revendication 1, à base d'une résine alkyde, acrylique, de mélamine, de polyuréthanne, époxydique ou de polyester, ou un mélange de ces résines.

**12.** Composés de Formule I

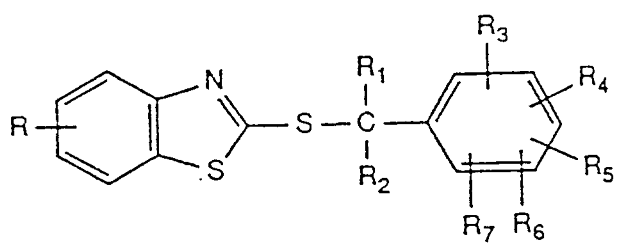

(I)

où R est hydrogène, un halogène ou un radical alkyle en $C_1$-$C_{12}$, halogénalkyle en $C_1$-$C_4$, alcoxy en $C_1$-$C_{12}$, alkylthio en $C_1$-$C_{12}$, phénylthio, benzylthio, alkylsulfonyle en $C_1$-$C_{12}$, phényle, $-NO_2$, -CN, -COOH, -COO(alkyle en $C_1$-$C_4$), -OH, $-NH_2$, $-NHR_8$, $-N(R_8)_2$, $-CONH_2$, $-CONHR_8$ ou $-CON(F_8)_2$,
$R_1$ est un hydrogène ou un radical alkyle en $C_1$-$C_{12}$, phényle, ou encore phényle substitué par des substituants halogéno, alkyle en $C_1$-$C_4$, alcoxy en $C_1$-$C_4$ ou $-NO_2$, ou encore pyridyle, thiényle ou furyle,
$R_2$ est un hydrogène ou un radical alkyle en $C_1$-$C_4$ ou phényle,
$R_3$ et $R_4$, indépendamment l'un de l'autre, sont chacun H ou un radical alkyle en $C_1$-$C_{20}$, alcoxy en $C_1$-$C_{20}$, $-NO_2$, -CN, -COOH, -COO(alkyle en $C_1$-$C_4$), phényle, halogéno, ou un groupe de formule II

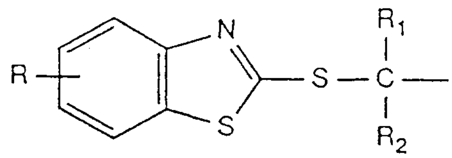

(II)

ou encore $R_3$ et $R_4$ forment ensemble un groupe -CH = CH-CH = CH-,

$R_5$, $R_6$ et $R_7$ sont chacun un hydrogène ou un halogène,

$R_8$ est un radical alkyle en $C_1$-$C_{12}$, alkyle en $C_3$-$C_{12}$ interrompu par un ou plusieurs oxygènes, cycloalkyle en $C_5$-$C_8$, benzyle, phényle, ou encore phényle substitué par des substituants halogéno, alkyle en $C_1$-$C_4$, alcoxy en $C_1$-$C_4$ ou $-NO_2$, ou encore $-N(R_8)_2$ est un groupe pyrrolidino, pipéridino ou morpholino, à l'exception des composés de Formule I dans laquelle R est un hydrogène ou un radical chloro, méthyle, méthoxy ou amino, $R_1$ et $R_2$ sont chacun un hydrogène, $R_3$ et $R_4$ sont chacun un hydrogène ou le chlore, et $R_5$, $R_6$ et $R_7$ sont chacun un hydrogène.

**13.** Composés selon la revendication 12 de Formule I, dans laquelle $R_1$ est un hydrogène ou un radical alkyle en $C_1$-$C_4$ ou phényle, et $R_2$ est un radical alkyle en $C_1$-$C_4$ ou phényle.

**14.** Composés selon la revendication 12 de Formule I, dans laquelle R est $-CF_3$, $-NO_2$, -COOH ou -COO-(alkyle en $C_1$-$C_4$).

**15.** Composés selon la revendication 12 de Formule I, dans laquelle $R_3$ et $R_4$, indépendamment l'un de l'autre, sont chacun un radical alkyle en $C_1$-$C_4$, alcoxy en $C_1$-$C_4$, $-NO_2$, -CN, -Br, -F ou un radical de Formule II, et $R_3$ et $R_4$ forment ensemble un radical -CH = CH-CH = CH-.